# EUROPEAN PATENT SPECIFICATION

(11) **EP 3 099 337 B1**
(45) Date of publication and mention of the grant of the patent: **06.04.2022**
(21) Application number: 14705025.6
(22) Date of filing: 29.01.2014
(51) Int. Cl.: A61L 2/02, A61L 9/16, B01D 39/00

(54) **TREATMENT DEVICE AND METHOD FOR DESTRUCTING MICRO-ORGANISMS IN GASEOUS OR LIQUID MEDIA**
BEHANDLUNGSVORRICHTUNG UND VERFAHREN ZUR ZERSTÖRUNG VON MIKROORGANISMEN IN GASFÖRMIGEN ODER FLÜSSIGEN MEDIEN
DISPOSITIF ET PROCÉDÉ DE TRAITEMENT POUR DÉTRUIRE DES MICRO-ORGANISMES DANS DES MILIEUX GAZEUX OU LIQUIDES

(43) Date of publication of application: 07.12.2016
(73) Proprietor: Norwegian Water Purification AS, 5012 Bergen (NO)
(72) Inventor: MASON, Dennis, 5105 Eisvåg/Åsane (NO)
(74) Representative: v. Bezold & Partner Patentanwälte - PartG mbB
(86) International application number: PCT/EP2014/000241
(87) International publication number: WO 2015/113575

(56) References cited:
- WO-A1-2004/080578
- WO-A1-2011/035104
- US-B1- 6 818 130

## Description

### Field of the invention

The present invention relates to a treatment device, which is adapted for destructing micro-organisms in a gaseous or liquid medium to be treated, in particular to a treatment device, which is configured for a through-flow of the medium through a treatment substance, wherein micro-organisms included in the medium are destructed (destroyed) by an interaction with the treatment substance. Furthermore, the present invention relates to a method of treating a gaseous or liquid medium for destructing micro-organisms included therein. Applications of the invention comprise water-cleaning, e. g. for disinfection purposes, environmental tasks or providing drinking water, or air flow cleaning, e. g. in an air conditioning device.

### Technical Background of the invention

Conventional treatment methods for removing micro-organisms in water typically are based on one of the following two approaches. Firstly, it is generally known to kill the microorganisms, e. g. with an UV irradiation or using chemical substances. The micro-organisms are destructed by the impact of the high-energy photons or the deadly effect of the chemical substance. Despite of the high efficiency of those conventional processes for killing the micro-organisms, they have substantial disadvantages in terms of technical complexity of an irradiation unit, which necessarily requires a power supply, and the contamination of the water with the chemical substance, respectively. Typically, chemical treatment of water does not provide drinking water, but rather requires further cleaning steps removing the chemical substance.

A second approach for removing micro-organisms from water is based on a filtering process. The water is flown through a filter substance, wherein the micro-organisms are adhered to the filter substance, e. g. by mechanical filtering and/or by a chemical adsorption to the filter substance. Examples of filtering techniques are described e. g. in US 6 818 130 B1, wherein microporous filter media, e. g. nonwoven microfiber glass web or charge-modified media, are used for capturing, killing or deactivating the micro-organisms. Another widely used technique is based on filtering water in a sand layer or in a glass granulate layer (GB 2 413 124 A). The glass granulate used in GB 2 413 124 A comprises glass pellets having a sub-angular shape, i.e. being rounded at the edges, and having a typical size above 1 mm. The glass pellets have a mechanical filtration effect and provide an adsorption on the surfaces thereof. They act as a surface catalytic filter media. Further filtering techniques are described in US 2007/0007213 A1 and US 2002/0104810 A1.

The conventional filter-based treatment methods have inherent disadvantages in terms of the need of a relatively high flow pressure as well as the requirement of periodical filter substance regeneration. Sand or glass granulate filters or even thin membrane filters require a relatively high pressure of the water when flowing through the filter material. Providing a complex pressure unit can be avoided only if extended layers of the filter material, e. g. in the earth, are used. The regeneration is necessary as the filter material will be clogged by the micro-organisms after a certain period of use. WO 2004/080578 A1 discloses a fluid purification system in particular including nanomaterials. The nanomaterials comprise nanotubes, which act on cells like a pin breaking a balloon.

### Objective of the Invention

The objective of the invention is to provide an improved treatment device, which is adapted for destructing microorganisms in a gaseous or liquid medium to be treated, wherein the treatment device is capable of avoiding disadvantages of conventional techniques. In particular, the disadvantages of both conventional destructing and filtering approaches are to be avoided. In particular, the objective of the invention is to provide a treatment device, which has a reduced complexity, has a high efficiency, does not require additional treatments and/or can be implemented with reduced or without consumption of electrical power. Furthermore, the objective of the invention is to provide an improved method of treating a gaseous or liquid medium for destructing micro-organisms included in the medium, avoiding disadvantages of conventional techniques.

### Summary of the Invention

The above objectives are solved correspondingly with a treatment device and a method of treating a gaseous or liquid medium, comprising the features of the independent claims, respectively. Advantageous embodiments and applications of the invention are defined in the dependent claims.

According to a first general aspect of the invention, the above objective is solved by a treatment device comprising a treatment substance. The treatment substance has an inner hollow structure allowing a through-flow of the medium to be treated. Furthermore, the treatment substance is contained in an enclosure accommodating the treatment substance and accommodating the flow of the medium through the inner hollow structure of the treatment substance. The enclosure is completely or partially filled with the treatment substance. The inner hollow structure of the treatment substance has internal edges which are cutting edges, as they have a capability of dissecting (fragmenting, cutting) the micro-organisms. According to a second general aspect of the invention, the above objective is solved by a method of treating a gaseous or liquid medium including micro-organisms, wherein the treatment device according to the above first aspect is used. According to the invention, the method comprises a step of flowing (pressing) the medium through the treatment substance. Furthermore, the method comprises a step of destructing the micro-organisms by a dissection thereof at the cutting edges of the inner hollow structure of the treatment substance.

Treating the medium generally comprises a destruction of living micro-organisms included in the medium, i. e. changing the micro-organisms such that they are no longer viable, by the mechanical interaction of the micro-organisms with the internal cutting edges of the treatment substance. In particular, the inventive treatment comprises a mechanical disinfection of the water or air. Thus the treatment substance is also called destruction mass.

The inventor has found, that flow forces (shear forces) are created in the medium by flowing it through the hollow structure. In particular by the effect of the enclosure encapsulating the treatment substance, the flow forces have a main direction parallel to the flow direction through the enclosure. Micro-organisms included in the medium hit the cutting edges of the hollow structure, where portions of the microorganisms, like flagella of bacteria, are cut and/or the cell membrane of the micro-organisms is destroyed. Additionally, the flow forces include turbulent forces, which are created by the effect of the cutting edges. The destructing grinding and milling effect of the edges is enhanced by the turbulent forces. The medium flow has the additional effect that the residual parts of the micro-organisms are carried through the treatment substance, i. e. the treatment substance as such substantially has no filtering effect.

As a main advantage of the invention, the micro-organism destruction and thus the removal of potentially living organisms from gaseous or liquid media is obtained by the flow of the medium through the treatment device only. The medium to be treated can be free of chemical substances disintegrating the micro-organisms. The destructing effect of the edges is supported by the inner pressure of micro-organism cells.

The provision of complex irradiation devices or chemical substances can be avoided. Furthermore, the invention does not require an additional shaking or vibrating device for creating flow forces in the medium. Furthermore, the treatment device can be operated with a long-term stability.

As a further advantage, the medium cleaning effect of the treatment device is not restricted to specific microorganisms, but rather obtainable with any living microorganisms, which can be included in gaseous or liquid media. Generally, the term "micro-organism" covers any single cell or multicellular organism having a size below 50 µm, including prokaryotic cells, like bacteria and archaea, or eukaryotic cells, like protozoa, fungi, algae, green algae or microscopic animals. Preferably, bacteria, in particular Escherichia coli and Legionella bacteria and/or algae are eliminated from water. The term "micro-organism" includes also viruses.

According to the invention, the treatment substance comprises particles arranged in the enclosure. The particles provide a packed bed, which forms the hollow structure of the treatment substance in the enclosure. The cutting edges of the hollow structure are provided by the surface shape of the particles. Using sharp edged particles as the treatment substance has particular advantages in terms of adapting the shape of the treatment substance to the inner space of the enclosure and the optional cleaning of the treatment substance after a certain period of use. Commercially available particles can be used, or they can be manufactured by milling treatment substance material.

Advantageously, the inventor has found that the particles can be made of various materials, including metal, glass, ceramic or diamond (diamond dust) or mixtures thereof, each having the edges on the particle surfaces. Preferred examples of metals comprise steel, copper or even aluminium. A preferred example of a ceramic is corundum, like the commercial product Korox (trade name).

Preferably, the particles of the treatment substance have a size above 10 µm, particularly preferred above 20 µm, e. g. 50 µm or larger. If the particles are smaller than 10 µm, there is a risk of clogging of the hollow structure by the micro-organisms. Furthermore, the particles preferably have a size below 500 µm, particularly preferred below 250 µm, e. g. 200 µm or smaller. With particles having a size above 500 µm, the destructing effect of the treatment substance could be reduced.

The micro-organisms included in the gaseous or liquid media are destroyed by intersecting the cell membrane or cell wall thereof by the effect of the cutting edges in the hollow structure. The stability and resistance of the treatment substance and the destructing effect can be improved if the treatment substance has a sufficient hardness for withstanding the flow forces and the impact of the micro-organisms. The inventor has found that the treatment substance which is used for manufacturing the particles, preferably has a Mohs hardness of at least 5, in particular at least 6. Additionally or alternatively, the hardness is selected such that the sharp edges of the particles are inflexible, and they have a fixed shape when subjected to the mechanical contact with the micro-organisms in the flowing media.

According to further preferred embodiments of the invention, the hollow structure of the treatment substance has at least one of the following geometric features. Preferably, inner spacings of the hollow structure have a width of at least 30 µm, particularly preferred at least 50 µm or at least 100 µm. Depending on the micro-organisms, larger spacings can be provided, e.g. 250 µm or larger. Above these limits, the effect of the flow forces is improved and the risk of clogging is reduced. Furthermore, the treatment substance is dimensioned such that the medium flow with residual parts of the microorganisms can pass the inner spacings.

According to the invention, the cutting edges have a blade shape. Advantageously, the blade shape can act like a knife. Preferably, the edges, in particular the blade lines thereof, have a radius of curvature below 1 µm, particularly preferred below 0,5 µm.

According to a variant of the invention, the particles of the treatment substance may comprise various materials, i.e. the treatment substance can comprise a mixture of different materials, shapes and/or sizes. Preferably, the particles are made by breaking the respective material, e. g. by crushing glass.

According to the invention, the medium to be treated is flown through the treatment device. To this end, the enclosure preferably has at least one input opening, which is adapted for applying the medium with a drive pressure to the treatment device, e. g. for supplying the medium with a pressure above atmospheric pressure. With a preferred example, the input opening is provided with a connector accommodating a tube, hose or funnel guiding the gaseous or liquid medium to be treated.

With a further preferred embodiment of the invention, the treatment device can be provided with a driving device, which is adapted for flowing the medium through the treatment substance, e. g. for applying the drive pressure to the medium. Advantageously, there are no particular requirements with regard to the flow rate. With preferred examples, the flow rate can be selected in a range of 0,1 ml/s to 10 ml/s, in particular 0,3 ml/s to 2 ml/s. Thus, various types of driving devices can be used, which can be selected in dependency on the particular application of the invention. The driving device can be driven electrically or manually. According to a first variant, the driving device comprises a screw conveyor. Advantageously, the screw conveyor can be included in the tube or hose connected with the treatment device. The screw conveyor can be driven with a motor or by hand. According to a second variant, a pump device can be provided, which creates the drive pressure in the medium to be treated. Again, an electrically driven or manually driven pump device can be used. A manually driven pump device is e. g. a bike pump or a balloon pump. As a further alternative or additional measure, a suction device can be provided at a downstream end of the treatment device. As a further particularly preferred embodiment of the invention, the drive device may comprise a hydrostatic pressure device. In particular for cleaning a liquid medium, the medium can be arranged in a container above the treatment device, wherein the container is connected via a pressure tight closed tubing with the enclosure. The medium flow is created by gravitational forces only.

According to a further advantageous embodiment of the inventive treatment device, a barrier element can be arranged at a downstream end of the treatment substance, in particular at an output opening of the enclosure. The barrier element is permeable for the medium to be treated and impermeable for the treatment substance. As an example, the retaining element may comprise a mesh having mesh openings smaller than the typical particle size of the treatment substance. The provision of the barrier element facilitates holding the treatment in the enclosure and applying an increased drive pressure for increasing the flow rate through the treatment substance.

Preferably, the barrier element additionally is adapted as a filter element. The filtering barrier element is permeable for the medium to be treated, but impermeable for the treatment substance and the residuals of the micro-organisms, in particular for the destroyed micro-organisms. Advantageously, the filtering barrier element provides a final filtering of the medium, thus increasing the cleaning effect of the treatment device.

As a further advantage of the invention, multiple applications of the treatment method are available. According to a preferred application, the medium to be treated comprises water, which is cleaned for providing drinking water. The invention is used for hygienic cleaning wastewater treatment. As a particularly important advantage of the invention, test experiments of the inventor have proven that by the inventive treatment of water including bacteria the micro-organisms can be removed to a degree that there is no health risk for a person drinking the water. With alternative applications of the invention, ballast water in a ship can be cleaned, or water in a fish farm can be cleaned.

The application of the invention is not restricted to liquid media, but also possible with gaseous media. As an example, an air flow of an air conditioning device can be flown through the treatment device for destroying micro-organisms included in the air flow.

Advantageously, the effect of the inventive treatment method can be improved if the medium is repeatedly flown through a treatment substance. A cascade of treatment devices can be provided. The cascade may comprise a parallel and/or serial multistage arrangement of treatment devices, wherein any further treatment device is coupled with the output opening of the enclosure of a foregoing treatment device, and/or a circulation arrangement, wherein the medium is redirected to the input opening of the enclosure after a treatment. The multistage arrangement may comprise e. g. two or more, e. g. 10, 20 or even more treatment devices.

Accordingly, a treatment plant including multiple treatment devices according to the above first aspect of the invention represents an independent subject of the invention.

According to a further advantageous variant of the inventive method, a step of regenerating the treatment substance can be provided. The treatment substance, in particular the particles can be removed from the enclosure and subjected to a washing process. Advantageously, the washing process can comprise washing in pure water. The addition of chemical washing media is not necessary.

As a further advantageous modification of the inventive method, the medium to be treated can be filtered before or after flowing through the treatment substance. Filtering removes larger particles or impurities from the medium, so that a clogging of the treatment substance is avoided.

### Brief description of the drawings

Further details and advantages of the invention are described in the following with reference to the attached drawings, which show in:
- Figure 1:: schematic illustrations of a first embodiment of the treatment device and treatment method according to the invention;
- Figures 2 to 4:: schematic illustrations of further embodiments of the treatment device according to the invention;
- Figure 5:: schematic illustrations of arrangements of multiple treatment devices; and
- Figure 6 and 7:: schematic illustrations of further embodiments of the treatment device according to the invention.

### Preferred embodiments of the invention

Preferred embodiments of the invention are described in the following with exemplary reference to a treatment device having a cylindrical, tubular enclosure. It is emphasized that the inventive treatment device can be manufactured with an enclosure having another shape, e. g. a cubic or flat shape. Furthermore, the implementation of the invention is not restricted to the removal of micro-organisms from water as described in the following. The media to be treated correspondingly may comprise watery solutions including microorganisms, or gases, in particular air. As an example, for cleaning air, the treatment device can be integrated into a circulation branch of an air conditioning system.

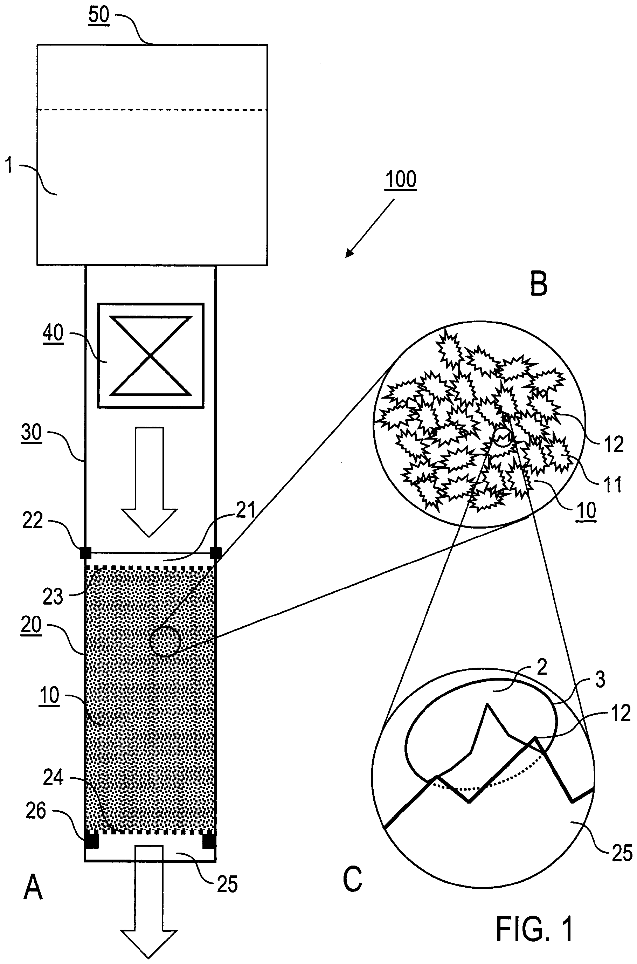

Figure 1 schematically illustrates features of the treatment device and the treatment method according preferred embodiments of the invention. The treatment device 100 comprises a treatment substance 10, which is arranged in an enclosure 20.

Furthermore, with the illustrated example, the treatment device 10 comprises a tube 30, a driving device 40 and a medium container 50 (Figure 1A). The container 50 may be a closed or open container being made of an inflexible or flexible material and accommodating the medium 1 to be treated. As an example, the medium container 50 may comprise a water reservoir for supplying drinking water. As a further example, the medium container 50 may comprise a bag, having a size of an infusion bag. This embodiment could be used to purify water volumes of 500 ml or less.

The treatment substance 10 comprises particles 11 with cutting edges 12 (Figures 1B, 1C). The particles 11 are schematically illustrated in the enlarged Figures 1B and 1C for illustrative purposes. It is emphasized that Figures 1B and 1C are schematic illustrations only. The shape and arrangement of the particles 11 can deviate from the illustrations in dependency on the particular particle material used. With a preferred example, the particles 11 are made of corundum having a cross sectional dimension of about 50 µm or glass having a cross sectional dimension of about 100 µm to 150 µm. The corundum particles have points and tips on the particle surface providing the cutting edges 12 of the treatment substance.

The enclosure 20 comprises a tubular body, which is made of e. g. plastic or metal, like steel. The enclosure 20 is configured for accommodating the flow of a medium 1 including micro-organisms 2 flowing along a longitudinal (axial) direction of the enclosure 20 (see large arrows in Figure 1A). The longitudinal length of the enclosure 20 and the treatment substance bed can be selected in dependency on the particular application, it is e. g. 20 cm. The inner diameter of the enclosure 20 is e. g. 2,5 cm.

At an upstream end of the enclosure 20, an input opening 21 with a connector 22 are provided. The connector 22 allows a screw connection with the tube 30 guiding the medium 1 to the treatment substance 10. Furthermore, the input opening 21 can be provided with a changeable input filter 23 which removes particles or impurities from the medium to be treated, so that a risk of clogging the treatment substance is reduced.

At a downstream end of the enclosure 20, a barrier element 24 is provided just before an output opening 25 of the enclosure 20. The barrier element 24, which is supported by a holding ring 26, retains the treatment substance 10 within the enclosure 20. As an example, a steel mesh is used as the barrier element 24.

The tube 30 is a solid or flexible connection between the medium container 50 and the enclosure 20. The schematically shown driving device 40, which comprises e. g. a pump, creates a drive pressure in the medium. The driving device 40 can be omitted, and the drive pressure can be generated as a hydrostatic pressure due to the gravitational force of the medium 1. Alternatively, the drive pressure can be generated by manually pumping the medium, e. g. by squeezing the medium container.

For treating the medium 1, the enclosure 20 with the treatment substance 10 is connected via the tube 30 to the medium reservoir 50. The driving device 40 is activated for flowing the medium 1 through the particles 11 of the treatment substance 10. Micro-organisms 2, as schematically shown in Figure 1C, hit sharp cutting edges 12 of the particles 11, so that the cell membrane 3 or cell wall of the micro-organism 2 is torn apart by the effect of the edges 12 and the flow. The remaining parts of the micro-organism are flown with the medium 1 through the inner space of the treatment substance 10 towards the downstream end thereof.

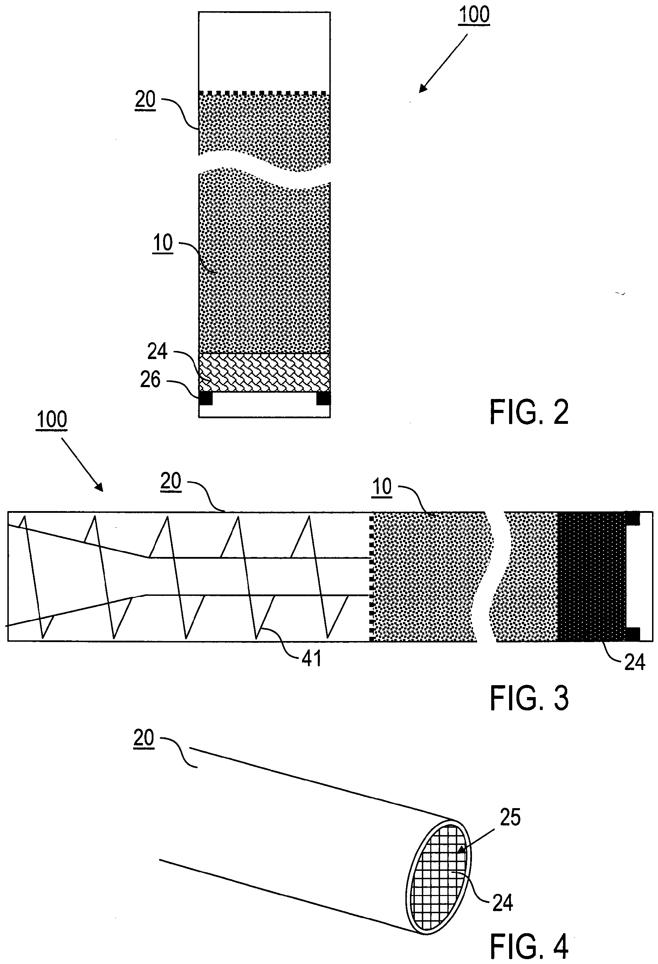

Figure 2 schematically illustrates a modified embodiment of the treatment device 100 comprising the treatment substance 10 accommodated in the enclosure 20. Further parts of the treatment device 100, like the connection with a reservoir, the optional driving device and the reservoir as such are not shown in Figure 2. For showing that the longitudinal length of the treatment device 100 can be chosen, the enclosure 20 is shown with a broken design. The difference of the embodiment of Figure 2 compared with the embodiment of Figure 1 is given by the design of the barrier element 24, which is arranged with the holding ring 26 at the downstream end of the enclosure 20. The barrier element 24 is a combined retaining and filter element. It is adapted for retaining the treatment substance 10 and collecting residuals of the destroyed microorganisms. The barrier element 24 comprises e. g. a porous material.

A further modification of a treatment device 100 according to the invention is schematically shown in Figure 3. With this embodiment, the enclosure 20 accommodates both the treatment substance 10 and the driving device comprising a screw conveyor 41. With the screw conveyor 41, the medium flow through the treatment device 10 is driven. It is one of the important results of the invention, that even a slow medium flow having a flow rate of e. g. 0,1 ml/s is sufficient to create destructing forces at the edges 12 of the particles 11 (see Figure 1C). As an alternative to Figure 3, the driving device may comprise a manually driven pump, like a bike pump.

As shown in Figure 3, the barrier element 24 may comprise a layer of activated carbon. The layer of activated carbon is permeable for the medium to be treated. Advantageously, inconvenient odor or taste can be removed from the medium using the activated carbon.

Figure 4 schematically shows a perspective view of the downstream end of the enclosure 20 with the output opening 25. The output opening 25 is closed by the barrier element 24, which is permeable for the liquid, e. g. water, only.

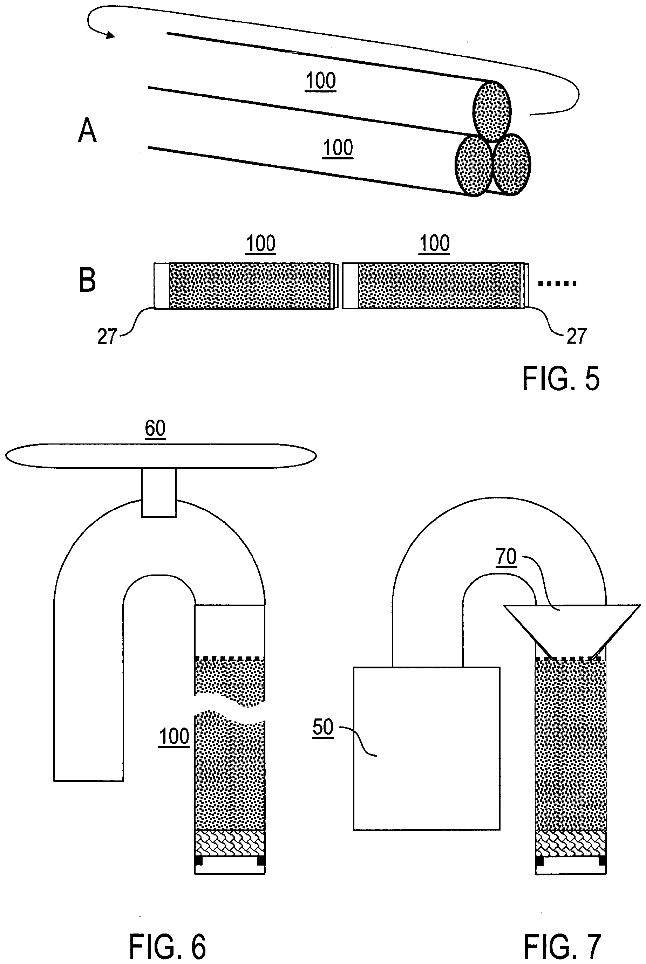

According to the invention, multiple treatment devices 100 can be combined, e. g. as a bundle, as shown in Figure 5A, and/or in series, as shown in Figure 5B. These embodiments may have advantages for adapting the cleaning capacity in dependency on the particular application of the inventive treatment method. Each of the treatment devices 100 within the bundle and/or serial connection can be structured as shown e. g. in the remaining figures. The arrow in Figure 5A illustrates that the multiple treatment devices 100 can be provided with a circulation arrangement. The medium can redirected to the input opening of the enclosure after a treatment. Figure 5B shows the treatment devices 100 before assembling them. Inner and outer windings 27 are provided at the axial ends of the enclosure 20, resp., so that the treatment devices 100 can be connected by screwing.

According to a further advantageous embodiment of the invention, which is schematically shown in Figure 6, the inventive treatment device 100 can be connected with a water tap 60. The water tap 60 is connected e. g. with a water supply system or a water reservoir (not shown). By the effect of the internal pressure of the water supply system, the water is driven through the treatment device 100. During the flow of the water through the treatment device 100, micro-organisms eventually contained in the water are killed. The water provided at the downstream end of the treatment device 100 has drinking quality. It can be used without additional cleaning steps.

As an alternative to Figure 6, the inventive treatment device 100 can be integrated at another location of a water supply, e. g. within a tube thereof.

Alternatively, water from a medium container 50 can be pumped to a funnel 70 connected with the input opening 21 of the treatment device 100, as shown in Figure 7. The medium to be treated flows through the treatment substance 10 by the effect of gravitational forces. During the flow, microorganisms contained in the medium are killed as described above.

### Test result

Test of the inventive treatment device according to Figure 1 were carried out by treating polluted sewage water from a commercial sewage purification facility. The polluted sewage water contained a high concentration of Escherichia coli. One litre of the water was placed under a pressure of 3 kg/cm and purified in the treatment device. Four different setups were used, with 250 ml of the water being forced through 1, 2, 4 and 8 cm longitudinal length of the treatment substance, respectively. After each test the system was cleaned using 1 litre of pure water before another 250 ml of sewage water was forced through the system. After the tests, the treatment substance and the entire treatment device was flushed with 1 litre of distilled water and samples were taken to record how many E. coli were still alive.

The polluted water contained 1 530 E. coli /100 ml, and the purification efficiency through 1 cm destruction mass was 96.2 %. Doubling the thickness of the mass increased the purification efficiency to 99.8 %, and a further doubling to 4 cm thickness increased the purification to 99.99 %. In the last setup, using 8 cm thickness of the destruction mass, no E. coli were recorded in the 4 samples of purified water. Furthermore, the inventive treatment device was investigated in further two separate ways: firstly, by "washing" through the purification system a litre of pure water immediately after use to see what living microorganisms could be washed out, and secondly, by examining the living microorganisms remaining in the destruction mass after the test. Both tests showed that the active part of the inventive treatment device is not a filter, but actually kills a large proportion of the bacteria.

The features of the invention disclosed in the above description, the drawings and the claims can be of significance both individually as well as in combination for the realization of the various embodiments.

## Claims

1. Treatment device (100), which is adapted for destructing micro-organisms (2) in a gaseous or liquid medium (1) to be treated, comprising:
- a treatment substance (10) having an inner hollow structure and being contained in an enclosure (20) accommodating a flow of the medium (1) through the inner hollow structure,
**characterized in that**
- the treatment substance (10) comprises particles (11) being arranged in the enclosure (20) and providing the inner hollow structure, said particles (11) having cutting edges (12) with a blade shape, and
- the cutting edges (12) are arranged for dissecting the micro-organisms (2) by a mechanical contact of the micro-organisms (2) with the cutting edges (12) of the hollow structure.

2. Treatment device according to claim 1, including at least one of the features
- the treatment substance (10) comprises at least one of metal, in particular steel, copper or aluminium, glass, ceramic and diamond particles, and
- the particles (11) have a size above 10 µm, in particular above 20 µm, and/or below 500 µm, in particular below 250 µm.

3. Treatment device according to one of the foregoing claims, including at least one of the features
- the treatment substance (10) has a Mohs hardness of at least 5, in particular at least 6,
- the treatment substance (10) has a hardness such that the cutting edges (12) have a fixed shape when subjected to the mechanical contact with the micro-organisms (2), and
- the cutting edges (12) have a radius of curvature below 1 µm, in particular at below 0,5 µm.

4. Treatment device according to one of the foregoing claims, wherein
- the enclosure (20) has at least one input opening (21), which is configured for applying a drive pressure to the medium to be treated.

5. Treatment device according to claim 4, wherein
- the input opening (21) comprises a connector (22) for connecting the enclosure (20) with a tube (30) guiding the medium (1) to be treated.

6. Treatment device according to one of the foregoing claims, further comprising
- a driving device (40), which is adapted for driving the flow of the medium (1) through the treatment substance (10).

7. Treatment device according to claim 6, wherein the driving device comprises
- a screw conveyor (41),
- a pump device (42),
- a suction device (43), or
- a hydrostatic pressure device (44).

8. Treatment device according to one of the foregoing claims, further comprising at least one of
- a medium container (50) accommodating the medium (1) to be treated and being in fluid communication with the enclosure (10),
- a barrier element (24) arranged at an output opening of the enclosure (20), wherein the barrier element is permeable for the medium (1) to be treated and impermeable for the treatment substance (10).

9. Treatment device according to claim 8, comprising the barrier element, wherein
- the barrier element (24) is impermeable for the destroyed micro-organisms (2), and/or
- the barrier element (24) includes activated carbon.

10. Treatment plant, which is adapted for dissecting micro-organisms (2) in a gaseous or liquid medium (1) to be treated, including multiple treatment devices according to one of the foregoing claims.

11. Method of treating a gaseous or liquid medium (1) including micro-organisms (2), wherein a treatment device (100) according to one of the claims 1 to 9 is used, comprising the steps of:
- flowing the medium (1) through the treatment substance (10), and
- dissecting the micro-organisms (2) by mechanically contacting the micro-organisms (2) with the internal cutting edges (12) of the hollow structure.

12. Method according to claim 11, including at least one of the features
- the medium (1) is pumped and/or sucked through the treatment substance (10), and
- the medium (1) is flowing through the treatment substance (10) under the effect of gravity.

13. Method according to one of the claims 11 to 12, wherein the treating of the medium (1) comprises at least one of
- cleaning water for providing drinking water,
- cleaning ballast water in a ship,
- cleaning water in a fish farm,
- cleaning an air flow created by an air conditioning device, and
- eliminating bacteria, in particular Escherichia coli and Legionella bacteria, and/or algea from water.

14. Method according to one of the claims 11 to 13, comprising at least one of the steps of
- repeated flowing the medium (1) through the treatment substance (20),
- regenerating the treatment substance (20) by a washing process, and
- filtering the medium (1) before or after flowing the medium (1) through the treatment substance (10).

## Patentansprüche

1. Behandlungsvorrichtung (100), die zur Zerstörung von Mikroorganismen (2) in einem zu behandelnden gasförmigen oder flüssigen Medium (1) ausgelegt ist, umfassend:
- eine Behandlungssubstanz (10), die eine innere Hohlstruktur aufweist und in einer Einfassung (20) enthalten ist, die eine Strömung des Mediums (1) durch die innere Hohlstruktur aufnimmt, **dadurch gekennzeichnet, dass**
- die Behandlungssubstanz (10) Partikel (11) umfasst, die in der Einfassung (20) angeordnet sind und die innere Hohlstruktur bilden, wobei die Partikel (11) Schneidkanten (12) mit einer Klingenform aufweisen, und
- die Schneidkanten (12) angeordnet sind, um die Mikroorganismen (2) durch einen mechanischen Kontakt der Mikroorganismen (2) mit den Schneidkanten (12) der Hohlstruktur zu zerlegen.

2. Behandlungsvorrichtung gemäß Anspruch 1, umfassend zumindest eines der Merkmale
- die Behandlungssubstanz (10) umfasst die Partikel aus zumindest einem von Metall, insbesondere Stahl, Kupfer oder Aluminium, Glas, Keramik und Diamant, und
- die Partikel (11) haben eine Größe über 10 µm, insbesondere über 20 µm, und/oder unter 500 µm, insbesondere unter 250 µm.

3. Behandlungsvorrichtung gemäß einem der vorhergehenden Ansprüche, umfassend zumindest eines der Merkmale
- die Behandlungssubstanz (10) weist eine Mohshärte von mindestens 5, insbesondere von mindestens 6 auf,
- die Behandlungssubstanz (10) weist eine solche Härte auf, dass die Schneidkanten (12) bei mechanischem Kontakt mit den Mikroorganismen (2) eine feste Form aufweisen, und
- die Schneidkanten (12) weisen einen Krümmungsradius geringer als 1 µm, insbesondere geringer als 0,5 µm, auf.

4. Behandlungsvorrichtung gemäß einem der vorhergehenden Ansprüche, wobei
- die Einfassung (20) mindestens eine Eingangsöffnung (21) aufweist, die zum Beaufschlagen des zu behandelnden Mediums mit einem Antriebsdruck ausgebildet ist.

5. Behandlungsvorrichtung gemäß Anspruch 4, wobei
- die Eingangsöffnung (21) einen Konnektor (22) zum Verbinden der Einfassung (20) mit einem das zu behandelnde Medium (1) führenden Schlauch (30) aufweist.

6. Behandlungsvorrichtung gemäß einem der vorhergehenden Ansprüche, ferner umfassend
- eine Antriebsvorrichtung (40), die zum Antreiben der Strömung des Mediums (1) durch die Behandlungssubstanz (10) eingerichtet ist.

7. Behandlungsvorrichtung gemäß Anspruch 6, wobei die Antriebsvorrichtung umfasst
- eine Förderschnecke (41),
- eine Pumpvorrichtung (42),
- eine Saugeinrichtung (43), oder
- eine hydrostatische Druckeinrichtung (44).

8. Behandlungsvorrichtung gemäß einem der vorhergehenden Ansprüche, ferner umfassend mindestens eines von
- einen Mediumbehälter (50), der das zu behandelnde Medium (1) aufnimmt und mit der Einfassung (10) in Fluidverbindung steht,
- ein Barriereelement (24), das an einer Ausgangsöffnung der Einfassung (20) angeordnet ist, wobei das Barriereelement für das zu behandelnde Medium (1) permeabel und für die Behandlungssubstanz (10) impermeabel ist.

9. Behandlungsvorrichtung gemäß Anspruch 8, umfassend das Barriereelement, wobei
- das Barriereelement (24) impermeabel für die zerstörten Mikroorganismen (2) ist, und/oder
- das Barriereelement (24) Aktivkohle enthält.

10. Behandlungsanlage, die zur Zerlegung von Mikroorganismen (2) in einem zu behandelnden gasförmigen oder flüssigen Medium (1) eingerichtet ist, umfassend mehrere Behandlungsvorrichtungen gemäß einem der vorhergehenden Ansprüche.

11. Verfahren zum Behandeln eines gasförmigen oder flüssigen Mediums (1), das Mikroorganismen (2) enthält, wobei eine Behandlungsvorrichtung (100) gemäß einem der Ansprüche 1 bis 9 verwendet wird, umfassend die Schritte:
- Durchströmen des Mediums (1) durch die Behandlungssubstanz (10), und
- Zerlegen der Mikroorganismen (2) durch mechanisches Kontaktieren der Mikroorganismen (2) mit den inneren Schneidkanten (12) der Hohlstruktur.

12. Verfahren gemäß Anspruch 11, umfassend zumindest eines der Merkmale
- das Medium (1) wird durch die Behandlungssubstanz (10) gepumpt und/oder gesaugt, und
- das Medium (1) strömt durch die Behandlungssubstanz (10) unter der Wirkung von Schwerkraft.

13. Verfahren gemäß einem der Ansprüche 11 bis 12, wobei die Behandlung des Mediums (1) zumindest eines umfasst von
- Reinigung von Wasser zur Bereitstellung von Trinkwasser,
- Reinigung von Ballastwasser in einem Schiff,
- Reinigen von Wasser in einer Fischzucht,
- Reinigen eines von einer Klimaanlage erzeugten Luftstroms, und
- Beseitigung von Bakterien, insbesondere Escherichia coli und Legionella-Bakterien, und/oder Algen aus Wasser.

14. Verfahren gemäß einem der Ansprüche 11 bis 13, umfassend zumindest eines der folgenden Schritte
- wiederholtes Durchströmen des Mediums (1) durch die Behandlungssubstanz (20),
- Regenerieren der Behandlungssubstanz (20) durch einen Waschvorgang, und
- Filtrieren des Mediums (1) vor oder nach dem Durchströmen des Mediums (1) durch die Behandlungssubstanz (10).

## Revendications

1. Dispositif de traitement (100), qui est adapté pour détruire des micro-organismes (2) dans un milieu (1) gazeux ou liquide à traiter, comprenant :
- une substance de traitement (10) ayant une structure creuse interne et étant contenue dans une enceinte (20) recevant un écoulement du milieu (1) à travers la structure creuse interne, **caractérisé en ce que**
- la substance de traitement (10) comprend des particules (11) étant agencées dans l'enceinte (20) et fournissant la structure creuse interne, lesdites particules (11) ayant des bords tranchants (12) avec une forme de lame, et
- les bords tranchants (12) sont agencés pour disséquer les microorganismes (2) par un contact mécanique des microorganismes (2) avec les bords tranchants (12) de la structure creuse.

2. Dispositif de traitement selon la revendication 1, incluant au moins l'une parmi les caractéristiques
- la substance de traitement (10) comprend au moins un élément parmi des particules de métal, en particulier d'acier, de cuivre ou d'aluminium, de verre, de céramique, et de diamant, et
- les particules (11) ont une taille supérieure à 10 µm, en particulier supérieure à 20 µm, et/ou inférieure à 500 µm, en particulier inférieure à 250 µm.

3. Dispositif de traitement selon l'une des revendications précédentes, incluant au moins l'une parmi les caractéristiques
- la substance de traitement (10) a une dureté Mohs d'au moins 5, en particulier d'au moins 6,
- la substance de traitement (10) a une dureté telle que les bords tranchants (12) ont une forme fixe lorsqu'ils sont soumis au contact mécanique avec le microorganisme (2), et
- les bords tranchants (12) ont un rayon de courbure inférieur à 1 µm, en particulier inférieur à 0,5 µm.

4. Dispositif de traitement selon l'une des revendications précédentes, dans lequel
- l'enceinte (20) a au moins une ouverture d'entrée (21), qui est configurée pour appliquer une pression d'entraînement au milieu à traiter.

5. Dispositif de traitement selon la revendication 4, dans lequel
- l'ouverture d'entrée (21) comprend un raccord (22) pour raccorder l'enceinte (20) à un tube (30) guidant le milieu (1) à traiter.

6. Dispositif de traitement selon l'une des revendications précédentes, comprenant en outre
- un dispositif d'entraînement (40), qui est adapté pour entraîner l'écoulement du milieu (1) à travers la substance de traitement (10).

7. Dispositif de traitement selon la revendication 6, dans lequel le dispositif d'entraînement comprend
- un convoyeur à vis (41),
- un dispositif de pompe (42),
- un dispositif d'aspiration (43), ou
- un dispositif à pression hydrostatique (44).

8. Dispositif de traitement selon l'une des revendications précédentes, comprenant en outre au moins l'un parmi
- un récipient de milieu (50) recevant le milieu (1) à traiter et étant en communication fluidique avec l'enceinte (10),
- un élément formant barrière (24) agencé au niveau d'une ouverture de sortie de l'enceinte (20), dans lequel l'élément formant barrière est perméable pour le milieu (1) à traiter et imperméable pour la substance de traitement (10).

9. Dispositif de traitement selon la revendication 8, comprenant l'élément formant barrière, dans lequel
- l'élément formant barrière (24) est imperméable pour les microorganismes (2) détruits, et/ou
- l'élément formant barrière (24) inclut du charbon actif.

10. Installation de traitement, qui est adaptée pour disséquer des microorganismes (2) dans un milieu (1) gazeux ou liquide à traiter, incluant de multiples dispositifs de traitement selon l'une des revendications précédentes.

11. Procédé de traitement d'un milieu (1) gazeux ou liquide incluant des microorganismes (2), dans lequel un dispositif de traitement (100) selon l'une des revendications 1 à 9 est utilisé, comprenant les étapes suivantes :
- écoulement du milieu (1) à travers à la substance de traitement (10), et
- dissection des microorganismes (2) par contact mécanique des microorganismes (2) avec les bords tranchants internes (12) de la structure creuse.

12. Procédé selon la revendication 11, incluant au moins l'une parmi les caractéristiques
- le milieu (1) est pompé et/ou aspiré à travers la substance de traitement (10), et
- le milieu (1) s'écoule à travers la substance de traitement (10) sous l'effet de la gravité.

13. Procédé selon l'une des revendications 11 à 12, dans lequel le traitement du milieu (1) comprend au moins l'un parmi
- le nettoyage d'eau pour fournir de l'eau potable,
- le nettoyage d'eau de ballast dans un bateau,
- le nettoyage d'eau dans une installation piscicole,
- le nettoyage d'un flux d'air créé par un dispositif de conditionnement d'air ; et
- l'élimination des bactéries, en particulier des bactéries Escherichia coli et Legionella, et/ou des algues de l'eau.

14. Méthode selon l'une des revendications 11 à 13, comprenant au moins l'une parmi les étapes suivantes
- l'écoulement répété du milieu (1) à travers la substance de traitement (20),
- la régénération de la substance de traitement (20) par un processus de lavage, et
- la filtration du milieu (1) avant ou après l'écoulement du milieu (1) à travers la substance de traitement (10).
